# FASCICULE DE BREVET EUROPEEN

(11) **EP 1 121 343 B1**
(45) Date de publication et mention de la délivrance du brevet: **28.08.2002**
(21) Numéro de dépôt: 99942996.2
(22) Date de dépôt: 17.09.1999
(51) Int. Cl.: C07C 249/00, C01B 21/16, C07C 251/88

(54) **PROCEDE DE PREPARATION D'HYDRAZINE**
VERFAHREN ZUR HERSTELLUNG VON HYDRAZIN
METHOD FOR PREPARING HYDRAZINE

(30) Priorité: 13.10.1998 FR 9812781
(43) Date de publication de la demande: 08.08.2001
(73) Titulaire: Atofina, 92800 Puteaux (FR)
(72) Inventeur: BRENGUER, Georges, F-65250 La Barthe de Neste (FR); JULLIN, Rémi, F-65300 Lannemezan (FR); RICARD, Jean-Philippe, F-64000 Pau (FR)
(86) Numéro de dépôt international: FR9902217
(87) Numéro de publication internationale: WO00021921

(56) Documents cités:
- EP-A- 0 399 866
- EP-A- 0 518 728

## Description

La présente invention a pour objet un procédé de préparation d'azine, et un procédé de préparation d'hydrazine le mettant en oeuvre.

La synthèse de l'hydrazine à partir d'ammoniac et d'eau oxygénée est décrite dans ULLMANN'S ENCYCLOPEDIA OF INDUSTRIAL CHEMISTRY (1989) Vol. A 13, pages 182-183.

Dans une première étape, on fait réagir dans un réacteur, de l'ammoniac, de l'eau oxygénée, et un réactif portant un groupe carbonyle, pour former une azine selon la réaction (I) :

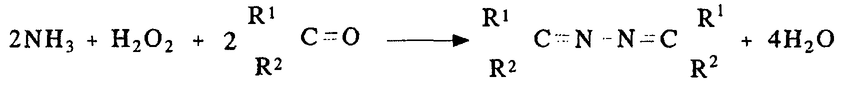

Dans ce schéma, R¹ et R², identiques ou différents, représentent un atome d'hydrogène ou un radical C₁-C₄ alkyle, à condition que l'un au moins de R¹ et R² soit différent d'un atome d'hydrogène, ou bien R¹ et R² forment ensemble avec l'atome de carbone auquel ils sont reliés un radical C₃-C₆ cycloalkyle.

Cette réaction est effectuée obligatoirement en présence d'un catalyseur (ou activateur) ou d'un mélange de catalyseurs compris dans une composition dénommée solution de travail. En fin de réaction, on sépare l'azine de la solution de travail. Cette dernière est alors régénérée, puis recyclée dans le réacteur de l'étape (I).

Dans une deuxième étape, l'azine est hydrolysée en hydrazine, selon la réaction (II) : 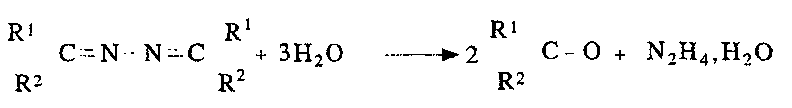 le réactif portant un groupe carbonyle est récupéré et recyclé dans le réacteur de l'étape (I).

Ce procédé a été décrit notamment dans les brevets US 3972878 et US 3972876.

On entend donc par solution de travail toute solution ou suspension aqueuse comprenant un catalyseur ou un mélange de catalyseurs capable de convertir un mélange d'ammoniac, d'eau oxygénée et d'un réactif portant un groupe carbonyle, en une azine.

Cette solution de travail est décrite dans les brevets EP 399866, EP 487160, EP 70155. Elle est par exemple constituée d'une solution aqueuse d'acétamide et d'acétate d'ammonium. L'acétate d'ammonium est formé dans le réacteur, notamment par réaction de l'acide acétique sur l'ammoniac. La solution de travail peut également être constituée d'une solution aqueuse d'acide cacodylique et de cacodylate d'ammonium.

La demande EP 0518728 donne à connaître un procédé de synthèse d'azine dans lequel la solution de travail et, plus généralement, tout courant entrant dans le réacteur de synthèse de l'azine, sont exempts de CO₂. Selon cette demande, la présence de CO₂ dans le procédé de synthèse résulte généralement de réactions indésirables du peroxyde d'hydrogène avec diverses impuretés organiques qui se produisent dans certaines étapes du procédé. Le CO₂ ainsi formé réagit à son tour avec le peroxyde d'hydrogène, ce qui entraîne une consommation excessive en H₂O₂, et une baisse du rendement en intermédiaire azine, et donc en hydrazine.

Pour éviter cet inconvénient, la demande EP 0518728 décrit un procédé de préparation d'azine intermédiaire, dans lequel :
a) on met en contact dans un réacteur, de l'ammoniac, de l'eau oxygénée, un réactif portant un groupe carbonyle, avec une solution de travail, puis
b) on sépare l'azine ainsi formée de la solution de travail, puis
c) on régénère la solution de travail en la portant à une température d'au moins 130°C, de telle manière que le CO₂ et l'essentiel de l'eau (formée durant la réaction de l'étape a) ou apportée par l'eau de dilution de l'eau oxygénée) sont éliminés sous la forme d'un courant aqueux, dénommé C1 dans la suite du présent texte, lequel comprend donc, à l'état dissous, outre le CO₂ (présent essentiellement sous forme de carbonate), une certaine quantité de l'ammoniac et du réactif portant un groupe carbonyle mis en oeuvre dans l'étape a), puis
d) on recycle la solution de travail, régénérée selon l'étape c), dans le réacteur de l'étape a).

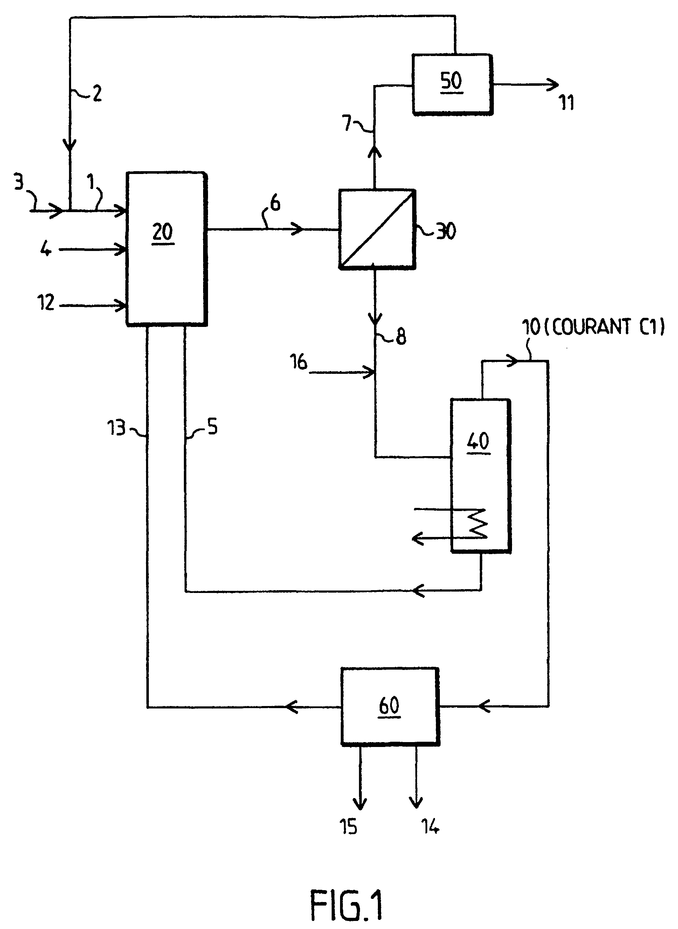

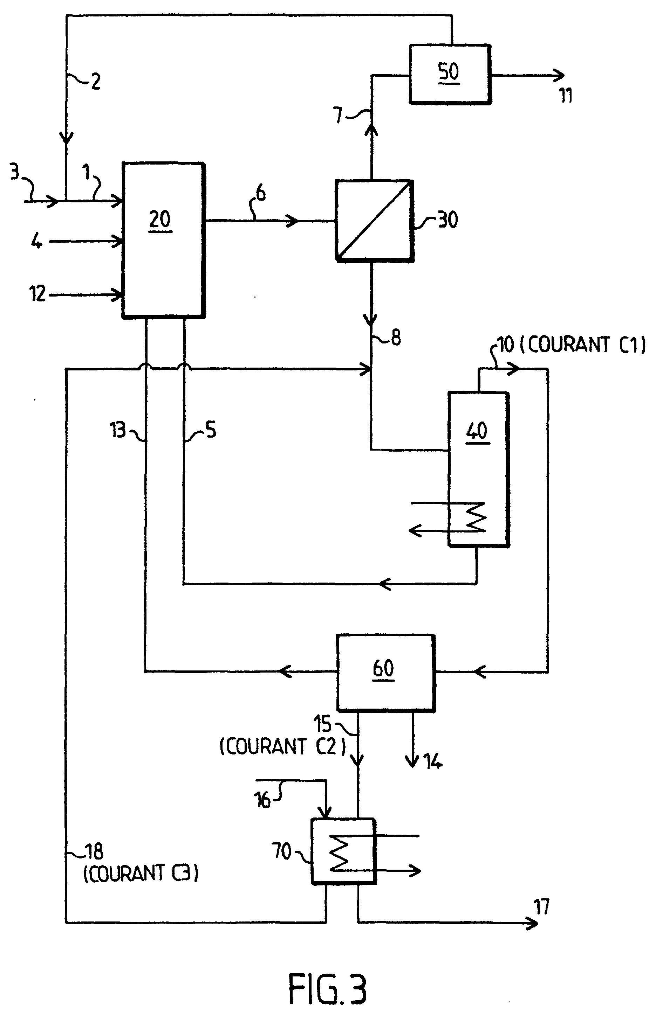

Le courant C1 comprend une certaine quantité d'ammoniac et de réactif portant un groupe carbonyle. Il ne peut cependant être recyclé directement dans le réacteur de synthèse de l'azine, précisément en raison de la présence d'une quantité notable de CO₂. La demande EP 0518728 préconise donc, pour économiser des quantités notables de réactifs utilisables dans le procédé, de compléter l'étape d) dudit procédé en traitant ce courant C1 de manière à en éliminer le CO₂ pour pouvoir ainsi recycler l'ammoniac et le réactif portant un groupe carbonyle qu'il contient, dans le réacteur de synthèse de l'azine. La figure 1/3 représente un dispositif adapté à la mise en oeuvre de ce procédé. Elle est décrite de façon détaillée plus loin dans le présent texte, dans la partie appropriée.

Un procédé de traitement du courant C1 approprié à l'élimination du CO₂, telle que mentionnée dans l'étape d), est décrit également par la demande EP 0518728.

Selon ce procédé, le courant C1 est traité tout d'abord dans une colonne de stripping, dont le courant de tête est refroidi dans un condenseur. Le NH₃ est recueilli à l'état gazeux, et la phase liquide obtenue est soumise à une séparation, dans un bac de décantation, entre la phase organique, riche en réactif portant un groupe carbonyle, et la phase aqueuse.

Le NH₃ et la phase organique ainsi obtenus sont recyclés vers le réacteur de synthèse de l'azine.

La phase aqueuse, qui contient tout le CO₂ présent dans le courant C1, est alimentée dans une 2ème colonne à distiller, de manière à recueillir :
- en tête de colonne, un courant aqueux exempt de CO₂ et contenant une certaine quantité d'ammoniac et de réactif portant un groupe carbonyle, qui est donc recyclé vers le réacteur de synthèse de l'azine,
- en pied de colonne, un courant aqueux, dénommé C2 dans la suite du présent texte, qui comprend la quasi-totalité du CO₂ présent dans C1 et encore de petites quantités d'ammoniac et de réactif portant un groupe carbonyle.

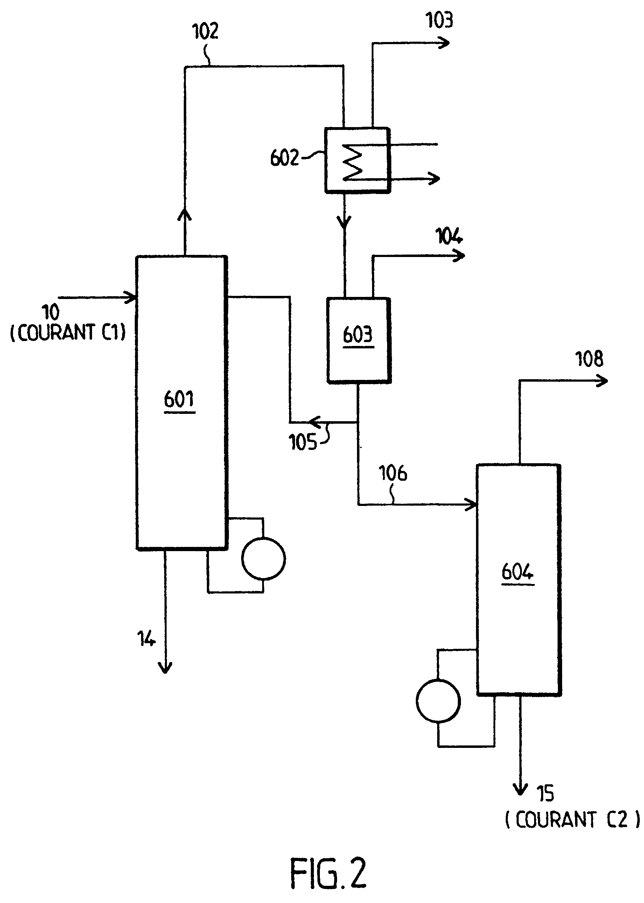

La figure 2/3 représente, pour rappel, le dispositif enseigné par EP 518728 pour le traitement du courant C1.

Dans les conditions pratiques de mise en oeuvre du procédé, le courant C2 comprend généralement de 0,1 à 20 % en poids de CO₂ (présent sous forme de carbonate à l'état dissous), de 1 à 15 % de NH₃, et de 0,1 à 10 % de réactif portant un groupe carbonyle.

Dans le présent texte, sauf indication contraire, les pourcentages indiqués pour les composants sous des pourcentages exprimés en poids.

La demande EP 0518728 ne donne aucun enseignement relatif au traitement à apporter au courant C2. Selon cette demande, il ne peut être recyclé vers le réacteur de synthèse de l'azine, puisqu'il contient du CO₂.

L'élimination d'un tel courant C2 pourrait être envisagé au moyen de techniques connues telles que l'incinération. Cependant, cette élimination s'accompagnerait nécessairement de dégagements dans l'atmosphère d'un mélange de composés du type NO ou NO₂, résultant de la combustion de NH₃. Or, il est souhaitable de limiter au maximum le rejet dans l'atmosphère de tels composés. De plus, cette élimination impliquerait encore la perte de petites quantités de réactifs utilisables dans le procédé, à savoir d'ammoniac et de réactif portant un groupe carbonyle, perte préjudiciable à l'économie du procédé.

La demande EP 0518728 suggère de procéder à l'élimination du CO₂ par un lavage à la soude pour retenir le CO₂ sous forme de solution aqueuse de carbonate de sodium, et laisser l'ammoniac, le réactif portant un groupe carbonyl, et la vapeur d'eau sous forme gazeuse.

Il a à présent été trouvé, de façon surprenante, un nouveau procédé de préparation de l'azine qui, contrairement à l'enseignement du brevet EP 0518728, fait appel à un acide pour le traitement du courant C2.

Un but de l'invention est donc de proposer un procédé de préparation d'azine dans lequel les rejets d'effluents dans l'environnement sont réduits.

Un autre but de l'invention est de proposer un procédé de préparation d'azine dans lequel les pertes en réactifs utiles pour la réaction sont diminuées.

Ces buts peuvent être atteints, en totalité ou en partie, par le procédé de préparation d'azine, objet de l'invention, qui est décrit ci-dessous.

L'invention a donc pour objet, en premier lieu, un procédé de préparation d'azine comprenant les étapes :
(i) dans laquelle, successivement :
   (a) on met en contact dans un réacteur, de l'ammoniac, de l'eau oxygénée, un réactif portant un groupe carbonyle, avec une solution de travail,
   (b) on sépare l'azine ainsi formée de la solution de travail,
   (c) on régénère la solution de travail en la portant à une température d'au moins 130°C, de telle manière que le CO₂ et l'essentiel de l'eau (formée durant la réaction de l'étape a) ou apportée par l'eau de dilution de l'eau oxygénée) sont éliminés sous la forme d'un courant aqueux C1, lequel comprend donc, à l'état dissous, outre le CO₂ (présent essentiellement sous forme de carbonate), une certaine quantité de l'ammoniac et du réactif portant un groupe carbonyle mis en oeuvre dans l'étape a),
   (d) on recycle la solution de travail régénérée selon l'étape c) dans le réacteur de l'étape a) ; et
(ii) dans laquelle, successivement :
   (a) on traite le dit courant C1 dans une colonne de stripping ;
   (b) on condense le courant de tête en séparant le NH₃ à l'état gazeux ;
   (c) on soumet à séparation la phase liquide issue de la condensation entre une phase organique, riche en réactif portant un groupe carbonyle, et une phase aqueuse comprenant tout le CO₂ présent dans C1 ;
   (d) on alimente la dite phase aqueuse dans une colonne à distiller d'où l'on recueille en tête un courant aqueux exempt de CO₂ et en pied un courant aqueux C2 comprenant la quasi-totalité du CO₂ présent dans C1,
caractérisé en ce que :
- on traite au moins une partie du courant C2 par une quantité d'acide telle que le pH dudit courant est amené à une valeur inférieure à 6,4, de préférence à 6 ; puis
- on recycle ladite partie (dénommée courant C3) ainsi traitée dans le réacteur de l'étape (i)(a).

Ce procédé est particulièrement avantageux en ce qu'il permet de récupérer l'essentiel des quantités de NH₃ et de réactif portant un groupe carbonyle présentes dans le courant C1, pour les réutiliser dans le réacteur de synthèse de l'azine. Il s'ensuit une économie appréciable. De plus, contrairement au traitement à la soude suggéré par EP 0518728, il permet d'éviter tout problème de rejet liquide ou solide dans l'environnement, le CO₂ étant éliminé par simple dégazage.

Le traitement à l'acide est habituellement réalisé à une température comprise entre 20 et 70°C, de préférence entre 40 et 60°C, et à une pression comprise entre 0,5 et 4 bars absolus, de préférence entre 0,5 et 2 bars absolus.

Le courant aqueux C2 mis en oeuvre a habituellement une teneur en CO₂ comprise entre 0,1 et 20 %, de préférence entre 0,5 et 5 %, une teneur en NH₃ comprise entre 1 et 15 %, de préférence entre 1 et 6 %, et une teneur en réactif portant un groupe carbonyle comprise entre 0,1 et 10 %, de préférence entre 0,1 et 5 %. Le pH du courant aqueux C2 est habituellement compris entre 8 et 12.

On préfère soumettre la totalité du courant C2 au traitement par l'acide.

On préfère également mettre en oeuvre le procédé selon l'invention en choisissant comme réactif portant un groupe carbonyle, la méthyléthylcétone. Dans ce cas l'azine correspondante est insoluble en solution aqueuse, ce qui facilite l'étape (i)(b) de séparation de l'azine de la solution de travail.

L'acide mis en oeuvre pour le traitement du courant aqueux C2 est avantageusement un acide de pKa inférieur à 6,3, de préférence inférieur à 5.

Selon une variante préférée du procédé selon l'invention, la solution de travail mise en oeuvre comprend une solution aqueuse d'acétamide et d'acide acétique. Dans ce cas, on préfère utiliser l'acide acétique pour le traitement du courant C2, comme acide de pKa inférieur à 6,3.

Selon une autre variante préférée du procédé selon l'invention, la solution de travail mise en oeuvre comprend une solution aqueuse d'acide cacodylique et de cacodylate d'ammonium. Dans ce cas, on préférera utiliser l'acide cacodylique pour le traitement du courant C2, comme acide de pKa inférieur à 6,3.

Dans tous les cas le traitement par neutralisation du courant C2 peut s'effectuer de manière très simple, plus particulièrement préférée, par utilisation du courant d'acide introduit dans le circuit de régénération de la solution de travail, pour compenser les pertes occasionnées par la circulation et le traitement de celle-ci. Le traitement du courant C2 s'effectuant à l'aide d'un réactif déjà mis en oeuvre dans le procédé, est par conséquent très avantageux en terme économique.

L'invention a également pour objet un procédé de préparation d'hydrazine, comprenant le procédé de préparation de l'azine, tel que décrit précédemment, et dans lequel l'azine séparée de la solution de travail conformément à l'étape (i)(b) du dit procédé est hydrolysée en hydrazine, le réactif portant un groupe carbonyle étant récupéré et recyclé dans le réacteur de synthèse de l'azine.

### Description détaillée des figures :

### Figure 1/3 :

La figure 1/3 représente un dispositif pour effectuer la synthèse de l'hydrazine à partir d'ammoniac, et d'eau oxygénée, selon un procédé mentionné dans EP 518728.

Dans le réacteur 20 on effectue la synthèse de l'azine. 1 représente l'alimentation en réactif portant un groupe carbonyle, constituée du recyclage venant du réacteur d'hydrolyse 50, et d'un appoint éventuel 3. 4 représente l'alimentation en NH₃, 12 l'alimentation en eau oxygénée, 5 représente le recyclage de la solution de travail. Par un tuyau 6, le produit de synthèse est amené dans le séparateur 30, qui produit en 7 l'azine brute et en 8 la solution de travail, qui contient aussi de l'ammoniac, un peu de cétone, l'eau qui s'est formée par réaction, et l'eau qui a été apportée par l'eau oxygénée puisque, pour des raisons de sécurité, on utilise l'eau oxygénée à une concentration d'au plus 70 % en poids dans l'eau.

Le séparateur 30 peut être un simple décanteur si l'azine est insoluble dans la solution de travail et dans l'eau de réaction, sinon on utilise une colonne à distiller. La solution de travail a pour fonction de catalyser la synthèse d'azines, et d'entraîner l'eau de réaction, et l'eau apportée avec l'eau oxygénée par le tuyau 8 jusqu'au dispositif 40.

Dans le dispositif 40, la solution de travail est portée à une température d'au moins 130°C, et de préférence comprise entre 150 et 250°C. On récupère en 10 un courant C1 contenant :
- du CO₂ sous forme de carbonate d'ammonium,
- l'eau formée par la réaction, et l'eau apportée par l'eau oxygénée,
- de l'ammoniac,
- du réactif porteur d'un groupe carbonyl.

On récupère en 5 la solution de travail régénérée, qui comprend de l'acétamide et de l'acide acétique, et qui est renvoyée dans le réacteur 20.

Le courant 10 (courant C1) est envoyé vers l'unité de traitement 60.

En sortie de cette unité 60, on obtient :
- un courant 13, correspondant à la juxtaposition d'un courant gazeux de NH₃ (courant 103 de la figure 2/3), d'un courant riche en MEK (courant 104 de la figure 2/3), et d'un courant aqueux contenant de petites quantités de NH₃ et de MEK (courant 108 de la figure 2/3) ; ce courant 13 est recyclé dans le réacteur 20 ;
- un courant d'eau 14,
- un courant 15 contenant le CO₂ présent dans le courant 10 et des petites quantités de NH₃ et de MEK.

Le courant d'introduction d'acide acétique 16 est prévu pour compenser les pertes occasionnées par la circulation et le traitement de la solution de travail.

En 50, I'azine est hydrolysée en hydrazine qu'on soutire en 11 et en 2 on recycle la cétone au réacteur 20.

### Figure 2/3 :

La figure 2/3 représente le détail du traitement effectué dans l'unité 60 de la figure 1/3. Ce traitement est conforme à un dispositif enseigné par EP 518728 pour le traitement du courant C1 correspondant au courant 10 des figures 1/3 et 2/3.

Le courant C1, tel que défini précédemment, arrive en tête d'une colonne de stripping 601 munie d'un rebouilleur. Le CO₂, dans 10, est sous forme de carbonate d'ammonium. On récupère en pied le courant d'eau 14, et un courant de tête 102 qu'on refroidit entre 30 et 50°C dans un condenseur 602. Une partie de NH₃ ne se condense pas et est recyclée par un courant 103 au réacteur de synthèse de l'azine. La phase liquide passe dans le bac 603.

Selon la solubilité du réactif portant un groupe carbonyle, on peut avoir une phase organique riche en ce réactif. C'est notamment le cas, pour la méthyéthylcétone. On recycle cette phase organique par un courant 104 vers le réacteur de synthèse de l'azine. La phase aqueuse contenue dans 603 contient tout le CO₂ (sous forme de carbonate d'ammonium) qui existait dans le courant C1 (courant 10 sur les figures). Une partie 105 de cette phase aqueuse est envoyée vers la colonne 601 comme reflux, le courant 106 est flashé sous basse pression dans une colonne 604.

La température de pied de la colonne 604 est maintenue entre 20 et 45°C par un rebouilleur, la pression est maintenue entre 8.10³ Pa (60 mmHg) et 12.10³ Pa (90 mmHg) absolus par une pompe à vide ou tout dispositif équivalent. Le courant 108 de tête contient l'ammoniac et le réactif portant un groupe carbonyle, et de l'eau. Ce courant est exempt de CO₂, et est donc recyclé vers le réacteur de synthèse de l'azine.

Le courant C2 est le courant 15, obtenu en pied de la colonne 604, qui contient tout le CO₂ (sous forme de carbonate d'ammonium) présent dans le courant C1, ainsi que de l'eau, et des petites quantités de NH₃, et de réactif portant un groupe carbonyle.

### Figure 3/3 :

Cette figure illustre un mode de réalisation du procédé selon l'invention. Dans cette figure, l'unité de traitement 60 est identique à celle représentée sur la figure 2/3. Seules les différences avec la figure 1/3 sont commentées ci-après.

Le courant 15 (courant C2) issu du pied de la colonne 604 est alimenté dans un réacteur 70 agité et maintenu à 45°C, dans lequel est introduit le courant 16 d'acide acétique introduit pour compenser les pertes occasionnées par la circulation et le traitement de la solution de travail.

Le courant 17 représente le courant de CO₂ éliminé par dégazage, et le courant 18 le courant aqueux C3 ne contenant sensiblement plus de CO₂ et contenant des petites quantités de NH₃ (sous forme d'acétate d'ammonium) et de MEK.

L' exemple suivant est donné à titre purement illustratif de la présente invention et ne doit en aucun cas être interprété comme en limitant la portée.

### Exemple :

On effectue la synthèse d'une azine, par réaction de la méthyléthylcétone (MEK), d'ammoniac, et d'eau oxygénée, en présence d'une solution de travail comprenant de l'acétamide et de l'acétate d'ammonium, conformément aux figures 2 et 3.

On obtient un courant 15 (courant C2), dont le débit est de 350 kg/h et qui comprend les composés suivants :

| | |
|---|---|
| CO₂ | 1,42 % (exprimé en poids équivalent molaire de carbonate) |
| NH₃ | 4 % |
| MEK | 1,5 % |

Le pH de ce courant est de 10,4.

Le courant 16 correspondant à l'introduction d'acide acétique à 75 % dans l'unité est de 150 kg/h. La totalité du courant 15 est introduit dans le réacteur d'acidification maintenu à une température de 45°C.

A la sortie du réacteur d'acidification, on obtient un courant 18 (courant C3), de débit 500 kg/h, dont le pH est de 5,5, dont la teneur en CO₂ est ramenée à 0,6 %, et qui comprend :

| | |
|---|---|
| acétate d'ammonium | 12 % |
| acide acétique | 13 % |
| MEK | 1 % |

Ce courant 18 est renvoyé vers le réacteur de synthèse de l'azine 20 par introduction dans le courant 8 du circuit de régénération de la solution de travail.

## Revendications

1. Procédé de préparation d'azine comprenant les étapes :
(i) dans laquelle, successivement :
a) on met en contact dans un réacteur, de l'ammoniac, de l'eau oxygénée, un réactif portant un groupe carbonyle, avec une solution de travail,
b) on sépare l'azine ainsi formée de la solution de travail,
c) on régénère la solution de travail en la portant à une température d'au moins 130°C, de telle manière que le CO₂ et l'essentiel de l'eau sont éliminés sous la forme d'un courant aqueux C1,
d) on recycle la solution de travail régénérée selon l'étape c) dans le réacteur de l'étape a) ; et
(ii) dans laquelle, successivement :
(a) on traite le courant C1 dans une colonne de stripping ;
(b) on condense le courant de tête en séparant le NH₃ à l'état gazeux ;
(c) on soumet à séparation la phase liquide issue de la condensation entre une phase organique, riche en réactif portant un groupe carbonyle, et une phase aqueuse comprenant tout le CO₂ présent dans C1 ;
(d) on alimente la dite phase aqueuse dans une colonne à distiller d'où l'on recueille en tête un courant aqueux exempt de CO₂ et en pied un courant aqueux C2 comprenant la quasi-totalité du CO₂ présent dans C1,
**caractérisé en ce que** :
- on traite au moins une partie du courant C2 par une quantité d'acide telle que le pH du dit courant est amené à une valeur inférieure à 6,4, de préférence à 6 ; puis
- on recycle la dite partie (dénommée courant C3) ainsi traitée dans le réacteur de l'étape (i)(a).

2. Procédé selon la revendication 1, **caractérisé en ce que** le traitement par l'acide est réalisé à une température comprise entre 20 et 70°C, de préférence entre 40 et 60°C, et à une pression comprise entre 0,5 et 4 bars absolus, de préférence entre 0,5 et 2 bars absolus.

3. Procédé selon l'une des revendications 1 ou 2, **caractérisé en ce que** le courant aqueux C2 a une teneur en CO₂ comprise entre 0,1 et 20 %, de préférence entre 0,5 et 5 %, une teneur en NH₃ comprise entre 1 et 15 %, de préférence entre 1 et 6 %, et une teneur en réactif portant un groupe carbonyle comprise entre 0,1 et 10 %, de préférence entre 0,1 et 5 %.

4. Procédé selon l'une des revendications 1 à 3, **caractérisé en ce que** on soumet la totalité du courant C2 au traitement par l'acide.

5. Procédé selon l'une des revendications 1 à 4, **caractérisé en ce que** le réactif portant un groupe carbonyle est la méthyléthylcétone.

6. Procédé selon l'une des revendications 1 à 5, **caractérisé en ce que** l'acide mis en oeuvre pour le traitement du courant C2 est un acide de pKa inférieur à 6,3, de préférence inférieur à 5.

7. Procédé selon l'une des revendications 1 à 6, **caractérisé en ce que** la solution de travail mise en oeuvre comprend une solution aqueuse d'acétamide et d'acide acétique, et **en ce qu'**on utilise l'acide acétique pour le traitement du courant C2.

8. Procédé selon l'une des revendications 1 à 6, **caractérisé en ce que** la solution de travail mise en oeuvre comprend une solution aqueuse d'acide cacodylique et de cacodylate d'ammonium et **en ce qu'**on utilise l'acide cacodylique pour le traitement du courant C2

9. Procédé selon l'une des revendications 7 ou 8, **caractérisé en ce que** le traitement du courant C2 s'effectue par utilisation du courant d'acide acétique ou cacodylique introduit dans le circuit de régénération de la solution de travail.

10. Procédé de préparation d'hydrazine, comprenant le procédé de préparation de l'azine, tel que défini dans l'une des revendications 1 à 9, et dans lequel l'azine séparée de la solution de travail conformément à l'étape (i)(b) est hydrolysée en hydrazine, le réactif portant un groupe carbonyle étant récupéré et recyclé dans le réacteur de synthèse de l'azine.

## Patentansprüche

1. Verfahren zur Azinherstellung, das die folgenden Schritte umfaßt:
einen Schritt (i), in dem in dieser Reihenfolge
a) Ammoniak, Wasserstoffperoxid und ein Reagens, das eine Carbonylgruppe trägt, in einem Reaktor mit einer Arbeitslösung in Kontakt gebracht werden,
b) das so gebildete Azin von der Arbeitslösung abgetrennt wird,
c) die Arbeitslösung regeneriert wird, indem sie auf eine Temperatur von mindestens 130 °C erwärmt wird, wodurch das CO₂ und der überwiegende Teil des Wassers in Form eines wäßrigen Stromes C1 entfernt werden,
d) die gemäß Schritt c) regenerierte Lösung in den Reaktor von Schritt a) zurückgeführt wird,
einen Schritt (ii), in dem in dieser Reihenfolge
(a) der Strom C1 in einer Strippersäule behandelt wird,
(b) der Kopfstrom kondensiert wird unter Abtrennung des NH₃ im gasförmigen Zustand,
(c) die flüssige Phase, die bei der Kondensation entsteht, aufgetrennt wird in eine organische Phase, die eine große Menge des Reagens enthält, das eine Carbonylgruppe trägt, und eine wäßrige Phase, die das gesamte CO₂ enthält, das in C1 enthalten ist,
(d) die wäßrige Phase in eine Destillationskolonne geleitet wird, von der am Kopf ein wäßriger Strom aufgefangen wird, der kein CO₂ enthält, und am Fuß ein wäßriger Strom aufgefangen wird, der praktisch das gesamte CO₂ enthält, das in C1 enthalten ist
**dadurch gekennzeichnet, daß**
- mindestens ein Teil des Stromes C2 mit einer solchen Menge einer Säure behandelt wird, daß der pH-Wert dieses Stromes auf einen Wert unter 6,4, vorzugsweise unter 6, gebracht wird und
- anschließend dieser so behandelte Teil (der als Strom C3 bezeichnet wird) in den Reaktor von Schritt (i)(a) zurückgeführt wird.

2. Verfahren nach Anspruch 1, **dadurch gekennzeichnet, daß** die Behandlung mit der Säure bei einer Temperatur im Bereich von 20 bis 70 °C, vorzugsweise 40 bis 60 °C, und einem Druck im Bereich von 0,5 bis 4 bar absolut, vorzugsweise 0,5 bis 2 bar absolut, durchgeführt wird.

3. Verfahren nach einem der Ansprüche 1 und 2, **dadurch gekennzeichnet, daß** der wäßrige Strom C2 einen CO₂-Gehalt im Bereich von 0,1 bis 20 %, vorzugsweise 0,5 bis 5 %, einen NH₃-Gehalt im Bereich von 1 bis 15 %, vorzugsweise 1 bis 6 %, und einen Gehalt an dem Reagens, das eine Carbonlygruppe trägt, im Bereich von 0,1 bis 10 %, vorzugsweise 0,1 bis 5 %, aufweist.

4. Verfahren nach einem der Ansprüche 1 bis 3, **dadurch gekennzeichnet, daß** der gesamte Strom C2 der Behandlung mit der Säure unterzogen wird.

5. Verfahren nach einem der Ansprüche 1 bis 4, **dadurch gekennzeichnet, daß** es sich bei dem Reagens, das eine Carbonylgruppe trägt, um Methylethylketon handelt.

6. Verfahren nach einem der Ansprüche 1 bis 5, **dadurch gekennzeichnet, daß** die Säure, die zur Behandlung des Stromes C2 eingesetzt wird, eine Säure mit einem pKa-Wert unter 6,3, vorzugsweise unter 5, ist.

7. Verfahren nach einem der Ansprüche 1 bis 6, **dadurch gekennzeichnet, daß** die eingesetzte Arbeitslösung eine wäßrige Lösung von Acetamid und Essigsäure umfaßt und daß Essigsäure für die Behandlung des Stromes C2 verwendet wird.

8. Verfahren nach einem der Ansprüche 1 bis 6, **dadurch gekennzeichnet, daß** die eingesetzte Arbeitslösung eine wäßrige Lösung von Kakodylsäure und Ammoniumkakodylat umfaßt und daß Kakodylsäure für die Behandlung des Stromes C2 verwendet wird.

9. Verfahren nach einem der Ansprüche 7 und 8, **dadurch gekennzeichnet, daß** die Behandlung des Stromes C2 unter Verwendung des Stromes von Essigsäure oder Kakodylsäure erfolgt, den man in den Kreislauf zur Regenerierung der Arbeitslösung einleitet.

10. Verfahren zur Hydrazinherstellung, das das wie in einem der Ansprüche 1 bis 9 definierte Verfahren zur Azinherstellung umfaßt und bei dem das von der Arbeitslösung gemäß Schritt (i)(b) abgetrennte Azin zum Hydrazin hydrolysiert wird, wobei das Reagens, das eine Carbonylgruppe trägt, zurückgewonnen und in den Reaktor zur Synthese des Azins zurückgeführt wird.

## Claims

1. Process for the preparation of azine comprising the stages:
(i) in which, successively:
(a) ammonia, hydrogen peroxide and a reactant carrying a carbonyl group are brought into contact, in a reactor, with a working solution,
(b) the azine thus formed is separated from the working solution,
(c) the working solution is regenerated by bringing it to a temperature of at least 130°C, so that the CO₂ and most of the water are removed in the form of an aqueous stream C1;
(d) the working solution, regenerated according to Stage c), is recycled in the reactor of Stage a); and
(ii) in which, successively:
(a) the stream C1 is treated in a stripping column;
(b) the top stream is condensed while separating the NH₃ in the gaseous state;
(c) the liquid phase resulting from the condensation is separated into an organic phase, rich in reactant carrying a carbonyl group, and an aqueous phase comprising all the CO₂ present in C1;
(d) the said aqueous phase is fed into a distillation column, from where an aqueous stream devoid of CO₂ is collected at the top and an aqueous stream C2 comprising virtually all the CO₂ present in C1 is collected at the bottom,
**characterized in that**:
- at least a portion of the stream C2 is treated with an amount of acid such that the pH of the said stream is brought to a value of less than 6.4, preferably less than 6; then
- the said portion (denoted stream C3) thus treated is recycled in the. reactor of Stage (i)(a).

2. Process according to Claim 1, **characterized in that** the treatment with the acid is carried out at a temperature of between 20 and 70°C, preferably between 40 and 60°C, and at a pressure of between 0.5 and 4 bar absolute, preferably between 0.5 and 2 bar absolute.

3. Process according to either of Claims 1 and 2, **characterized in that** the aqueous stream C2 has a CO₂ content of between 0.1 and 20%, preferably between 0.5 and 5%, an NH₃ content of between 1 and 15%, preferably between 1 and 6%, and a content of reactant carrying a carbonyl group of between 0.1 and 10%, preferably between 0.1 and 5%.

4. Process according to one of Claims 1 to 3, **characterized in that** the whole of the stream C2 is subjected to the treatment with the acid.

5. Process according to one of Claims 1 to 4, **characterized in that** the reactant carrying a carbonyl group is methyl ethyl ketone.

6. Process according to one of Claims 1 to 5, **characterized in that** the acid employed in the treatment of the stream C2 is an acid with a pKₐ of less than 6.3, preferably of less than 5.

7. Process according to one of Claims 1 to 6, **characterized in that** the working solution employed comprises an aqueous solution of acetamide and of acetic acid and **in that** acetic acid is used in the treatment of the stream C2.

8. Process according to one of Claims 1 to 6, **characterized in that** the working solution employed comprises an aqueous solution of acodylic acid and of ammonium cacodylate and **in that** cacodylic acid is used in the treatment of the stream C2.

9. Process according to either of Claims 7 and 8, **characterized in that** the treatment of the stream C2 is carried out by use of the stream of acetic acid or of cacodylic acid introduced into the circuit for the regeneration of the working solution.

10. Process for the preparation of hydrazine, comprising the process for the preparation of azine as defined in one of Claims 1 to 9, in which the azine separated from the working solution in accordance with Stage (i)(b) is hydrolysed to hydrazine, the reactant carrying a carbonyl group being recovered and recycled in the reactor for the synthesis of the azine.
